# EUROPEAN PATENT APPLICATION

(11) **EP 4 248 953 A1**
(43) Date of publication of application: **27.09.2023**
(21) Application number: 21894038.5
(22) Date of filing: 19.11.2021
(51) Int. Cl.: A61K 9/70, A61K 31/475, A61K 47/18, A61K 47/38, A61K 47/32, A61K 47/44, A61K 47/40, A61P 19/02, A61P 29/00

(54) **BRUCINE GEL PLASTER AND PREPARATION METHOD AND USE THEREOF**

(30) Priority: 20.11.2020 CN 202011305433
(71) Applicant: Beijing Increasepharm Corporation Limited, Beijing 102200 (CN)
(72) Inventor: HU, Jie, Beijing 102200 (CN); XUE, Chunmei, Beijing 102200 (CN); ZHAO, Qian, Beijing 102200 (CN); FANG, Mifen, Beijing 102200 (CN)
(74) Representative: Cabinet Laurent & Charras
(86) International application number: PCT/CN2021/131901
(87) International publication number: WO 2022/105891

(57) **Abstract**

Provided are a brucine gel plaster and a preparation method therefor. The brucine gel plaster comprises, calculated by weight percentage, 0.3%-2% of brucine, 3%-10% of a framework material, 20%-50% of a humectant and a co-solvent, 0.1%-2% of a crosslinking agent and a crosslinking regulator, and the balance of water, as well as a backing layer and an anti-adhesive layer. The brucine gel plaster uses simple medicinal excipients, does not use a transdermal enhancer, has good skin compatibility, is moisturizing and breathable, is non-irritating to the skin and mucous membranes, has a large drug load, and can be applied repeatedly. The plaster is used for treating or ameliorating related diseases requiring anti-inflammation and analgesia such as osteoarthritis, rheumatic arthritis and rheumatoid arthritis.

## Description

### TECHNICAL FIELD

The present disclosure belongs to the field of pharmaceutical preparations, and in particular relates to a brucine gel plaster and its preparation method and use.

### BACKGROUND

Osteoarthritis (OA) is a degenerative joint disease, clinically manifested as joint pain, swelling, and stiffness, among other symptoms. The main symptom of arthritis is joint swelling and pain, and the most important pathological feature of osteoarthritis is the damage of articular cartilage. Strychnos nux-vomica L. is bitter and warm in nature; it is very poisonous. It has the effects of removing obstruction in collaterals, relieving pain, dissipating stagnation, and reducing swelling. It can be used for bruises, swelling and pain caused by fracture, obstinate rheumatic numbness, numbness and paralysis, and so on. Strychnos nux-vomica L. medicinal material has a history of being used for dissipating stagnation, reducing swelling, removing obstruction in collaterals, relieving pain, and treating arthritis for thousands of years, and is clinically used mostly in compound prescriptions. The main active ingredients of Strychnos nux-vomica L. are total alkaloids, which include brucine, strychnine, vomicine, novacine, strychnoside, icajine, and the like. Brucine is the medicinal ingredient of the Chinese herbal medicine Strychnos nux-vomica L. when the latter is used in the treatment of arthritis. Modem pharmacological studies have proved that brucine not only has anti-inflammatory and analgesic activities, but is also capable of promoting the proliferation of chondrocytes and inhibiting the early apoptosis of chondrocytes. Strychnine basically has no anti-inflammatory and analgesic effects and is the main toxic component of Strychnos nux-vomica L.

The brucine-related preparations that have been on the market are all compound prescriptions, and as a result of containing strychnine, they have a narrow therapeutic window. No formulation containing brucine alone has been available on the market until now.

Brucine has an oil-water partition coefficient (logP) of between 1 and 2 and could be favorably percutaneously absorbed. There have been existing technologies that develop topical compound preparations of brucine, such as gels, gel patches, and patches making use of pressure-sensitive adhesives, but all of them require the addition of a transdermal enhancer such as azone (which has a strong penetration enhancing effect) in the prescription in order to improve the transdermal effect of the active ingredients. Azone has an extremely strong skin-penetration promoting effect, but because of its high lipophilicity, during long-term use, azone accumulates in the skin for a long time, destroys the stratum corneum, causes irreversible skin damage, and has high irritancy.

The present disclosure provides a brucine gel plaster and a preparation method thereof. The brucine gel plaster of the present disclosure, without using a transdermal enhancer, is capable of achieving good transdermal absorption effect, and enables greatly improved safety in clinical application when used to treat osteoarthritis and rheumatic arthritis.

### SUMMARY

To deal with the defects in the prior art, the present disclosure aims to provide a novel brucine gel plaster that is safe, sustained-release, and excellent in transdermal effect, as well as its preparation method and use. Moreover, the brucine gel plaster provided by the present disclosure has good anti-inflammatory and analgesic effects as shown by drug efficacy tests, could be prepared by a simple process that is easy to operate, thus capable of realizing industrial production.

A first aspect of the present disclosure is to provide a brucine gel plaster comprising 0.3 wt% to 2 wt% of brucine, 3 wt% to 10 wt% of a framework material, 20 wt% to 50 wt% of a humectant and a cosolvent, 0.1 wt% to 2 wt% of a cross-linking agent and a cross-linking regulator, and water as the balance; as well as a backing layer and an anti-adhesive layer.

Preferably, the brucine gel plaster comprises 0.3 wt% to 1 wt% of brucine, 4 wt% to 8 wt% of a framework material, 25 wt% to 40 wt% of a humectant and a cosolvent, 0.3 wt% to 1 wt% of a cross-linking agent and a cross-linking regulator, and water as the balance; as well as a backing layer and an anti-adhesive layer.

In the brucine gel plaster, the framework material is one or more selected from the group consisting of carbomer, sodium alginate, gum arabic, tragacanth, gelatin, starch, xanthan gum, polyacrylic acid (sodium polyacrylate), polyvinyl alcohol, povidone K90, povidone K30, sodium carboxymethylcellulose, polyvinylpyrrolidone, HPMC, alginic acid and its sodium salt, polyacrylic acid cross-linked resin and a partially neutralized product of polyacrylic acid (NP-600, NP-700, or NP-800). Preferably, the framework material is one or more selected from the group consisting of a partially neutralized product of polyacrylic acid (NP-600, NP-700, or NP-800), sodium polyacrylate, povidone K90, and sodium carboxymethylcellulose.

In the brucine gel plaster, the humectant and the cosolvent is one or more selected from the group consisting of ethanol, urea, olive oil, polysorbate 80, polysorbate 60, propylene glycol, glycerin, DMSO, diethylene glycol monoethyl ether, polyethylene glycol 400, eucalyptus oil, cyclodextrin, and sorbitol. Preferably, the humectant and the cosolvent is one or a combination of two or more selected from the group consisting of glycerin, propylene glycol, DMSO, polysorbate 80, polyethylene glycol 400, and cyclodextrin.

In the brucine gel plaster, each of the cross-linking agent and the cross-linking regulator is one or more selected from the group consisting of aluminum hydroxide, aluminum glycinate, aluminum trichloride, aluminum sulfate, alum, a complex aluminum salt, tartaric acid, citric acid, malic acid, edetic acid, and a sodium salt thereof. Preferably, each of the cross-linking agent and the cross-linking regulator is one or more selected from the group consisting of aluminum glycinate, aluminum hydroxide, tartaric acid, citric acid, edetic acid, and a sodium salt thereof.

In the brucine gel plaster, the backing layer is selected from materials well known to those skilled in the art, such as non-woven fabric and rayon.

In the brucine gel plaster, the anti-adhesive layer is selected from materials well known to those skilled in the art, such as embossed polypropylene film, polyethylene film, anti-adhesive paper, plastic thin film, and hard gauze.

In some embodiments of the first aspect of the present disclosure, the brucine gel plaster may also comprise 0.05 wt% to 0.075 wt% of an antioxidant. The antioxidant is one or more selected from the group consisting of anhydrous sodium sulfite, sodium metabisulfite, sodium bisulfite, sodium thiosulfate, tert-butyl p-hydroxyanisole, dibutylphenol, vitamin C, vitamin C palmitate, edetic acid and its sodium salt.

The brucine gel plaster may also comprise 0.01 wt% to 0.3 wt% of a bacteriostatic agent, and preferably 0.1 wt% to 0.2 wt% of a bacteriostatic agent. The bacteriostatic agent is one or more selected from the group consisting of chlorobutanol, ethylparaben, benzyl alcohol, methylparaben, butylparaben, sorbic acid and its potassium salt, menthol, and benzoic acid and its sodium salt.

The brucine gel plaster may also comprise 0.1 wt% to 10 wt% of a filler, and preferably 0.1 wt% to 5 wt% of a filler. The filler is one or more selected from the group consisting of kaolin, diatomaceous earth, bentonite, zinc oxide, titanium dioxide, and micro-powder silica gel. Preferably, the filler is one or more selected from the group consisting of kaolin, titanium dioxide, and micro-powder silica gel.

The present disclosure provides a preparation method of a brucine gel plaster, and the brucine gel plaster may be prepared by any one of Method 1 to Method 3.

Method 1, comprising the following steps: (1) preparing a blank matrix that comprises a humectant and a cosolvent, a bacteriostatic agent, a framework material, a cross-linking agent and a cross-linking regulator, an antioxidant, and water in a prescribed amount; (2) dissolving brucine in a cosolvent and mixing with the blank matrix to obtain a brucine paste; and (3) applying the brucine paste on a backing layer and attaching an anti-adhesive layer to obtain a brucine gel plaster.

Method 2, comprising the following steps: (1) preparing a brucine organic phase that comprises a humectant and a cosolvent, brucine, a bacteriostatic agent, a framework material, a cross-linking agent and a cross-linking regulator, and an antioxidant; (2) preparing an aqueous matrix that comprises a cross-linking agent and a cross-linking regulator and water; (3) mixing the brucine organic phase with the aqueous matrix to obtain a brucine paste; and (4) applying the brucine paste on a backing layer and attaching an anti-adhesive layer to obtain a brucine gel plaster.

Method 3, comprising the following steps: (1) preparing an organic phase matrix that comprises a humectant, a bacteriostatic agent, a framework material, a cross-linking agent and a cross-linking regulator, and an antioxidant; (2) preparing a brucine aqueous phase that comprises brucine, a cross-linking agent and a cross-linking regulator, a cosolvent, and water; (3) mixing the organic phase matrix with the brucine aqueous phase to obtain a brucine paste; and (4) applying the brucine paste on a backing layer and attaching an anti-adhesive layer to obtain a brucine gel plaster.

Method 4, comprising the following steps:
(1) preparing an organic phase matrix that comprises a humectant, a framework material, and a cross-linking agent and a cross-linking regulator; (2) preparing a brucine aqueous phase that comprises brucine, a cross-linking regulator, and water; (3) mixing the organic phase matrix with the brucine aqueous phase to obtain a brucine paste; and (4) applying the brucine paste on a backing layer and attaching an anti-adhesive layer to obtain a brucine gel plaster, wherein the organic phase matrix in step (1) further optionally comprises a bacteriostatic agent and an antioxidant.

All the excipients in the above methods are adjusted in accordance with the actual prescribed amount and type.

The present disclosure provides use of a brucine gel plaster in the preparation of a medicament for knee osteoarthritis.

Unless otherwise specified, the terms in the present disclosure are defined as follows.

The term "blank matrix" refers to all components except brucine in a brucine paste.

The term "organic phase" refers to a part that is immiscible with water and mainly composed of organic compounds.

The term "aqueous phase" refers to a part mainly composed of water.

The term "framework material" refers to a substance that serves as a supporting framework of a product, serves as a reservoir of a medicament, produces viscosity, and serves to preserve water and moisture.

The term "cosolvent" refers to a substance that facilitates the dissolution and release of a main drug.

The term "humectant" refers to a substance that delays moisture loss and improves the stability of the product.

The term "crosslinking agent" refers to a substance that enables a product to have a microscopic three-dimensional structure and improves the stability of the product.

The term "cross-linking regulator" refers to a substance that improves the stability of a product and keeps the drug in an optimal dissolution state.

Compared with the prior art, the present disclosure has the following beneficial effects.
1. Oral administration of brucine produces high toxicity and side effects in the treatment of arthritis. A topical plaster of brucine has been developed, thereby avoiding toxicity caused by oral administration, improving the bioavailability of the drug, and bringing about an improved therapeutic effect.
2. The prepared brucine gel plaster uses simple pharmaceutical excipients; without using a strongly irritating transdermal enhancer such as azone, it achieves an excellent transdermal effect, has good skin compatibility, preserves moisture and has air permeability, shows no irritation to skin and mucous membranes, enables high drug load, and could be applied repeatedly.

### DETAILED DESCRIPTION

Embodiments of the present disclosure will be described in detail below by way of examples, but it is understandable to those skilled in the art that the following examples are only used to illustrate the present disclosure and should not be considered as limiting the scope of the present disclosure. Those methods in the examples for which no specific conditions are indicated are carried out under conventional conditions or under conditions suggested by manufacturers. The reagents or instruments used herein for which no manufacturer is indicated are all conventional products that are commercially available. The inventors of the present disclosure has obtained the technical solutions of the present disclosure through screening process of formulation and preparation process for which the inventors has made considerable creative efforts.

### Preliminary experiments for formulation screening

The optimal formulations and processes of the present disclosure were determined through a large number of formulation screening and process research. Some screening processes of formulation were as shown in Table 1.

**Table 1 Screening process of formulation and process of brucine gel plaster**

| No. | Pre-Experiment 1 | Pre-Experiment 2 | Pre-Experiment 3 | Pre-Experiment 4 | Pre-Experiment 5 | Pre-Experiment 6 | Pre-Experiment 7 | Pre-Experiment 8 | Pre-Experiment 9 |
|---|---|---|---|---|---|---|---|---|---|
| Glycerin | 20.00% | 20.00% | 20.00% | 20.00% | 20.00% | 20.00% | 20.00% | 20.00% | 20.00% |
| Propylene glycol | 5.00% | 5.00% | 5.00% | 5.00% | / | 5.00% | 5.00% | 5.00% | 5.00% |
| NP700 | 6.00% | 6.00% | 6.00% | 6.00% | / | 3.00% | 4.00% | 6.00% | 6.00% |
| Aluminu m glycinate | 0.20% | / | 0.20% | 0.20% | / | 0.20% | 0.30% | 0.30% | 0.30% |
| Aluminu m chloride | / | 0.20% | / | / | / | / | / | / | / |
| Tartaric acid | 0.20% | 0.20% | 0.20% | 0.20% | / | 0.20% | 0.30% | 0.50% | 0.50% |
| Brucine | 0.50% | 0.50% | 0.50% | 0.50% | 0.50% | 0.50% | 0.50% | 0.50% | 0.50% |
| Disodium edetate | 0.30% | 0.30% | 0.30% | / | / | 0.30% | 0.30% | 0.30% | 0.30% |
| Benzyl alcohol | | / | 1.00% | / | / | / | / | / | / |
| CMC-Na | / | / | / | / | 3.00% | 3.00% | / | / | / |
| PVPK90 | / | / | / | / | / | / | 2.50% | / | / |
| Kaolin | / | / | / | / | 5.00% | / | / | / | / |
| Gelatin | / | / | / | / | 3.00% | / | / | / | / |
| PEG400 | / | / | / | / | 10.00% | / | / | 10.00% | 10.00% |
| Preparatio n method | Method 3 | Method 3 | Method 3 | Method 3 | Method 2 | Method 3 | Method 3 | Method 1 | Method 2 |
| Characteri sties | Appropriate viscosity, easy to apply, flat plaster surface | Excessively fast crosslinking , uncontrollab le crosslinking speed of paste, unfavorable to uniform mixing of the paste and its application | Benzyl alcohol is not well compatible with brucine and is easily degraded | Short curing time, unfavorable to mass production | Poor cohesion, easy to penetrate the backing layer, making its characteristi cs greatly influenced by temperature | Appropriate viscosity, easy to apply, flat plaster surface | Appropriate viscosity, easy to apply, flat plaster surface | Appropriate viscosity, easy to apply, flat plaster surface | Appropriate viscosity, easy to apply, flat plaster surface |

The balance in each of the formulations was water. As indicated by the above formulations, substituting CMC-Na, gelatin or the like for partially neutralized sodium polyacrylate led to instability in the characteristics of the resulting plaster, while a gel plaster with stable characteristics was obtained from partially neutralized sodium polyacrylate, a cross-linking agent and a cross-linking regulator (as main components) together with a suitable cosolvent, a humectant, and a framework material. The preparation method could be selected according to the kind of excipients, and the gel plasters resulting from these three methods had stable and good characteristics.

### Example 1

| Formulation | Amount (g) |
|---|---|
| Brucine | 6 |
| Glycerin | 400 |
| Propylene glycol | 160 |
| NP-700 | 120 |
| Aluminum glycinate | 6 |
| Ethylparaben | 2 |
| Disodium edetate | 4 |
| Tartaric acid | 6 |
| Water | 1296 |
| Backing layer | Non-woven fabric |
| Anti-adhesive layer | Embossed polypropylene film |

The preparation method was as follows.

(1) An organic phase matrix was prepared by dissolving ethylparaben in propylene glycol, followed by adding thereto glycerin, NP-700, disodium edetate, and aluminum glycinate and mixing the resulting mixture evenly; (2) a brucine aqueous phase was prepared by dissolving tartaric acid and brucine in water and mixing the resulting mixture evenly, and a brucine paste was prepared by adding the brucine aqueous phase to the organic phase matrix and stirring the mixture evenly; (3) a brucine gel plaster was prepared by applying the brucine paste on a backing layer and attaching an anti-adhesive layer.

### Example 2

| Formulation | Amount (g) |
|---|---|
| Brucine | 12 |
| Glycerin | 400 |
| Propylene glycol | 100 |
| Ethanol | 100 |
| NP-600 | 60 |
| CMC-Na | 60 |
| Aluminum glycinate | 6 |
| Tartaric acid | 6 |
| Menthol | 20 |
| Disodium edetate | 4 |
| Water | 1232 |
| Backing layer | Non-woven fabric |
| Anti-adhesive layer | Embossed polypropylene film |

The preparation method was as follows.

(1) An organic phase matrix was prepared by dissolving menthol in ethanol, followed by adding thereto propylene glycol, glycerin, NP-600, CMC-Na, disodium edetate, and aluminum glycinate and mixing the resulting mixture evenly; (2) a brucine solution was prepared by dissolving brucine and tartaric acid in water and mixing the resulting mixture evenly, and a brucine paste was prepared by adding the brucine solution to the organic phase matrix and stirring the mixture evenly; (3) a brucine gel plaster was prepared by applying the brucine paste on a backing layer and attaching an anti-adhesive layer.

### Example 3

| Formulation | Amount (g) |
|---|---|
| Brucine | 12 |
| Glycerin | 400 |
| Propylene glycol | 100 |
| Ethanol | 100 |
| PEG400 | 100 |
| Sodium polyacrylate | 140 |
| Aluminum glycinate | 6 |
| Tartaric acid | 10 |
| Disodium edetate | 4 |
| water | 1128 |
| Backing layer | Non-woven fabric |
| Anti-adhesive layer | Embossed polypropylene film |

The preparation method was as follows.

(1) A brucine organic phase was prepared by dissolving brucine in a mixed solution of PEG400 and ethanol, followed by adding thereto propylene glycol, glycerin, sodium polyacrylate, disodium edetate, and aluminum glycinate and mixing the resulting mixture evenly; (2) an aqueous phase solution was prepared by dissolving tartaric acid in water and mixing the resulting mixture evenly, and a brucine paste was prepared by adding the aqueous phase solution to the brucine organic phase and stirring the mixture evenly; (3) a brucine gel plaster was prepared by applying the brucine paste on a backing layer and attaching an anti-adhesive layer.

### Example 4

| Formulation | Amount (g) |
|---|---|
| Brucine | 20 |
| Glycerin | 400 |
| Propylene glycol | 100 |
| DMSO | 160 |
| NP-800 | 60 |
| Kaolin | 60 |
| CMC-Na | 60 |
| Aluminum glycinate | 4 |
| Tartaric acid | 4 |
| Disodium edetate | 4 |
| water | 1128 |
| Backing layer | Non-woven fabric |
| Anti-adhesive layer | Embossed polypropylene film |

The preparation method was as follows.

(1) A brucine organic phase was prepared by dissolving brucine in DMSO, followed by adding thereto propylene glycol, glycerin, NP-800, CMC-Na, kaolin, disodium edetate, and aluminum glycinate and mixing the resulting mixture evenly; (2) an aqueous phase solution was prepared by dissolving tartaric acid in water and mixing the resulting mixture evenly, and a brucine paste was prepared by adding the aqueous phase solution to the brucine organic phase and stirring the mixture evenly; (3) a brucine gel plaster was prepared by applying the brucine paste on a backing layer and attaching an anti-adhesive layer.

### Example 5

| Formulation | Amount (g) |
|---|---|
| Brucine | 12 |
| NP-700 | 80 |
| Glycerin | 400 |
| Propylene glycol | 100 |
| Ethylparaben | 1.6 |
| Povidone K90 | 50 |
| Aluminum glycinate | 6 |
| Tartaric acid | 6 |
| Disodium edetate | 6 |
| Polysorbate 80 | 60 |
| Water | 1278.4 |
| Backing layer | Non-woven fabric |
| Anti-adhesive layer | Embossed polypropylene film |

The preparation method was as follows.

(1) An organic phase matrix was prepared by dissolving ethylparaben in propylene glycol, followed by adding thereto glycerin, NP-700, disodium edetate, aluminum glycinate, and povidone K90 and mixing the resulting mixture evenly; (2) an aqueous phase solution of brucine was prepared by dissolving brucine, tartaric acid and polysorbate 80 in water and mixing the resulting mixture evenly, and a brucine paste was prepared by adding the aqueous phase solution of brucine to the organic phase matrix and stirring the mixture evenly; (3) a brucine gel plaster was prepared by applying the brucine paste on a backing layer and attaching an anti-adhesive layer.

### Example 6

| Formulation | Amount (g) |
|---|---|
| Brucine | 10 |
| Glycerin | 400 |
| Propylene glycol | 160 |
| NP-700 | 100 |
| Aluminum hydroxide | 6 |
| Ethylparaben | 2 |
| Disodium edetate | 4 |
| Tartaric acid | 10 |
| Eucalyptus oil | 40 |
| Polyvinyl alcohol | 60 |
| Water | 1208 |
| Backing layer | Non-woven fabric |
| Anti-adhesive layer | Embossed polypropylene film |

The preparation method was as follows.

(1) An organic phase matrix was prepared by dissolving ethylparaben in propylene glycol, followed by adding thereto glycerin, eucalyptus oil, NP-700, disodium edetate, aluminum hydroxide, and polyvinyl alcohol and mixing the resulting mixture evenly; (2) an aqueous phase solution of brucine was prepared by dissolving brucine and tartaric acid in water and mixing the resulting mixture evenly, and a brucine paste was prepared by adding the aqueous phase solution of brucine to the organic phase matrix and stirring the mixture evenly; (3) a brucine gel plaster was prepared by applying the brucine paste on a backing layer and attaching an anti-adhesive layer.

### Example 7

| Formulation | Amount (g) |
|---|---|
| Brucine | 24 |
| Glycerin | 400 |
| Propylene glycol | 160 |
| Sodium polyacrylate | 80 |
| Aluminum glycinate | 6 |
| Ethylparaben | 2 |
| Disodium edetate | 4 |
| Tartaric acid | 6 |
| HPMC | 80 |
| Polysorbate 60 | 100 |
| Water | 1138 |
| Backing layer | Non-woven fabric |
| Anti-adhesive layer | Embossed polypropylene film |

The preparation method was as follows.

(1) An organic phase matrix was prepared by dissolving ethylparaben in propylene glycol, followed by adding thereto glycerin, sodium polyacrylate, disodium edetate, HPMC, and aluminum glycinate and mixing the resulting mixture evenly; (2) an aqueous phase solution of brucine was prepared by dissolving brucine, tartaric acid and polysorbate 60 in water and mixing the resulting mixture evenly, and a brucine paste was prepared by adding the aqueous phase solution of brucine to the organic phase matrix and stirring the mixture evenly; (3) a brucine gel plaster was prepared by applying the brucine paste on a backing layer and attaching an anti-adhesive layer.

### Example 8

| Formulation | Amount (g) |
|---|---|
| Brucine | 10 |
| Glycerin | 400 |
| DMSO | 160 |
| Propylene glycol | 100 |
| NP-800 | 60 |
| Aluminum glycinate | 6 |
| Ethylparaben | 2 |
| Disodium edetate | 4 |
| Tartaric acid | 6 |
| Povidone K90 | 60 |
| Kaolin | 60 |
| β-cyclodextrin | 40 |
| Water | 1092 |
| Backing layer | Non-woven fabric |
| Anti-adhesive layer | Embossed polypropylene film |

The preparation method was as follows.

(1) An organic phase matrix was prepared by dissolving ethylparaben in propylene glycol, followed by adding thereto DMSO, glycerin, NP-800, povidone K90, kaolin, aluminum glycinate, and disodium edetate and mixing the resulting mixture evenly; (2) an aqueous phase solution of brucine was prepared by dissolving brucine, tartaric acid and β-cyclodextrin in water and mixing the resulting mixture evenly, and a brucine paste was prepared by adding the aqueous phase solution of brucine to the organic phase matrix and stirring the mixture evenly; (3) a brucine gel plaster was prepared by applying the brucine paste on a backing layer and attaching an anti-adhesive layer.

### Example 9

| Formulation | Amount (g) |
|---|---|
| Brucine | 16 |
| Glycerin | 400 |
| Propylene glycol | 160 |
| NP-700 | 80 |
| Aluminum glycinate | 6 |
| Ethylparaben | 2 |
| Disodium edetate | 4 |
| Tartaric acid | 6 |
| Urea | 40 |
| Micro-powder silica gel | 80 |
| Titanium dioxide | 2 |
| Water | 1204 |
| Backing layer | Non-woven fabric |
| Anti-adhesive layer | Embossed polypropylene film |

The preparation method was as follows.

(1) An organic phase matrix was prepared by dissolving ethylparaben in propylene glycol, followed by adding thereto glycerin, NP-700, disodium edetate, aluminum glycinate, micro-powder silica gel, and titanium dioxide and mixing the resulting mixture evenly; (2) an aqueous phase solution of brucine was prepared by dissolving brucine, tartaric acid and urea in water and mixing the resulting mixture evenly, and a brucine paste was prepared by adding the aqueous phase solution of brucine to the organic phase matrix and stirring the mixture evenly; (3) a brucine gel plaster was prepared by applying the brucine paste on a backing layer and attaching an anti-adhesive layer.

### Example 10

| Formulation | Amount (g) |
|---|---|
| Brucine | 12 |
| Glycerin | 400 |
| Propylene glycol | 160 |
| Oleic acid | 50 |
| NP-600 | 80 |
| Aluminum glycinate | 10 |
| Ethylparaben | 2 |
| Disodium edetate | 4 |
| Citric acid | 6 |
| CMC-Na | 60 |
| Sodium alginate | 100 |
| Kaolin | 60 |
| Titanium dioxide | 2 |
| Water | 1054 |
| Backing layer | Non-woven fabric |
| Anti-adhesive layer | Embossed polypropylene film |

The preparation method was as follows.

An organic phase matrix was prepared by dissolving ethylparaben in propylene glycol, followed by adding thereto glycerin, oleic acid, NP-600, CMC-Na, sodium alginate, kaolin, disodium edetate, titanium dioxide, and aluminum glycinate and mixing the resulting mixture evenly; (2) an aqueous phase solution of brucine was prepared by dissolving brucine and citric acid in water and mixing the resulting mixture evenly, and a brucine paste was prepared by adding the aqueous phase solution of brucine to the organic phase matrix and stirring the mixture evenly; (3) a brucine gel plaster was prepared by applying the brucine paste on a backing layer and attaching an anti-adhesive layer.

### Experimental example 1: Characteristic study of the brucine gel plaster preparation of the present disclosure

Visual observation of the physical characteristics of the gel plaster preparations according to Examples 1-10 showed that they had a flat, smooth and uniform appearance, and their formability, initial adhesion and film residual amount all met the requirements.

### Experimental example 2: Release rate study of the brucine gel plaster preparation of the present disclosure

The "Paddle over Disk" method was adopted. According to Approach 2 of the "Paddle over Disk" method (Method 4) in the section "0931 Dissolution and Release Rate Determination Methods" under the part "General Rules" in Volume IV of the China Pharmacopoeia 2015, the specific method was as follows.

900 ml of the dissolution medium (4 % sodium chloride solution) was respectively measured, added to dissolution cups, and pre-warmed to 32 °C±0.5 °C. The brucine gel plasters prepared in Example 1, Example 2, Example 3, Example 4, Example 5, Example 6, Example 7, Example 8, Example 9, and Example 10 were fixed on net dishes with their sides to be tested for dissolution facing up, and were kept as flat as possible. The net dishes were then placed horizontally in the lower part of the dissolution cups and allowed to be parallel to the rotating surface of the bottom of the paddles with a distance therebetween of 25 mm ± 2 mm. The paddles rotated at a speed of 50 rpm. Samples were taken periodically, a proper amount of the dissolution liquid was aspirated, and the same volume of the dissolution medium at a temperature of 32 °C ± 0.5 °Cwas supplemented in time. The results were shown in Table 2.

**Table 2 Results of release rate experiment on brucine gel plasters**

| Time (h) | 0 | 0.5 | 1 | 3 | 5 | 8 |
|---|---|---|---|---|---|---|
| Example 1 (%) | 0 | 26.39 | 38.07 | 65.78 | 80.14 | 90.52 |
| Example 2 (%) | 0 | 40.33 | 56.84 | 86.35 | 96.31 | 100.53 |
| Example 3 (%) | 0 | 35.17 | 43.23 | 73.96 | 84.87 | 98.05 |
| Example 4 (%) | 0 | 42.11 | 54.49 | 84.87 | 95.97 | 99.78 |
| Example 5 (%) | 0 | 43.21 | 60.57 | 88.17 | 98.39 | 100.04 |
| Example 6 (%) | 0 | 42.43 | 55.02 | 86.84 | 95.29 | 99.86 |
| Example 7 (%) | 0 | 41.67 | 58.09 | 87.14 | 95.68 | 100.63 |
| Example 8 (%) | 0 | 38.07 | 57.78 | 87.02 | 95.54 | 100.02 |
| Example 9 (%) | 0 | 43.57 | 59.08 | 87.29 | 94.09 | 100.35 |
| Example 10 (%) | 0 | 25.93 | 37.29 | 68.74 | 82.16 | 93.04 |

When the brucine gel plasters provided by the present disclosure were subjected to *in-vitro* release rate experiment, except that the brucine gel plasters prepared in Examples 1 and 10 had a slightly slower release rate as compared with other Examples, the brucine gel plasters prepared in the rest of the Examples had no significant difference in terms of release rate. All Examples of the present disclosure achieved an 8-hour cumulative release rate of greater than 90 %, indicating that good effects were achieved.

### Experimental example 3: In-vitro release study of the brucine gel plaster preparation of the present disclosure

The Franz diffusion cell method was adopted, and the *ex-vivo* abdominal skin of rat was used as the barrier. The test samples were the brucine gel plasters prepared in Example 2, Example 4, Example 5, Example 6, Example 7, Example 8, Example 9, and the commercially available indomethacin cataplasm (manufactured by Nipro Pharmaceuticals Co. Ltd., with H20181060 as the imported drug registration code) as the sample for comparative group. The specific method was as follows.

Rats were sacrificed by cervical dislocation. The hair on their abdomen and surrounding areas was removed with a razor, and the whole rat skin of the depilated part was peeled off with a dissecting scissor. After being washed, the skin was naturally fixed on a modified Franz diffusion cell such that the skin surface faced the drug supply chamber and the inner layer of the skin was in exact contact with the receiving liquid. The gel plaster from which the anti-adhesive layer had been removed was attached to the skin surface. The receiving liquid was a PBS buffer (pH=7.4) that had been preheated to 37 °C. The temperature of the water bath in the receiving chamber was 37 °C, and the magnetic stirring speed was 500 rpm. Samples were taken at 1, 2, 4, 6, 8, and 24 hours, respectively. The receiving liquid was all taken out, and an equal amount of fresh receiving liquid was added. The results were shown in Table 3.

**Table 3 Results of in-vitro transdermal experiment on brucine gel plasters**

| Time (h) | 0 | 1 | 2 | 4 | 6 | 8 | 24 |
|---|---|---|---|---|---|---|---|
| Example 2 (µg) | 0 | 0.78 | 2.15 | 6.38 | 10.45 | 14.89 | 76.37 |
| Example 4 (µg) | 0 | 0.19 | 0.56 | 2.01 | 3.42 | 5.68 | 49.15 |
| Example 5 (µg) | 0 | 0.21 | 0.78 | 3.52 | 6.89 | 15.25 | 80.02 |
| Example 6 (µg) | 0 | 0.23 | 0.56 | 3.08 | 6.56 | 12.74 | 55.81 |
| Example 7 (µg) | 0 | 0.20 | 0.77 | 3.19 | 6.78 | 14.15 | 70.61 |
| Example 8 (µg) | 0 | 0.19 | 0.69 | 2.77 | 5.89 | 10.96 | 52.63 |
| Example 9 (µg) | 0 | 0.10 | 0.63 | 2.98 | 6.32 | 11.05 | 56.62 |
| Comparative Example (commercially available indomethacin cataplasm) | 0 | 0.27 | 0.74 | 1.98 | 3.48 | 5.33 | 32.36 |

The brucine gel plasters provided by the present disclosure did not employ a transdermal enhancer that has strong penetration-enhancing effect such as azone to enhance the transdermal effect of the active ingredient. The commercially available indomethacin cataplasm in the comparative example, however, contained azone. In the *in-vitro* transdermal experiment, the experimental results showed that all the Examples of the present disclosure achieved a 24-hour cumulative transdermal amount (mass) higher than that of the medicament in comparative group.

### Experimental example 4: Pharmacodynamic experiment

The formulations in Table 3 were subjected to screening research via the preliminary drug efficacy experiment on osteoarthritis, and the brucine gel plasters of Example 2, Example 5 and Example 7 had better drug efficacy for treating osteoarthritis. Therefore, the brucine gel plasters of Example 2, Example 5 and Example 7 were subjected to formal pharmacodynamics research for osteoarthritis. The specific experimental method and results were as follows.

### 4.1 Experimental animals and drugs

### 4.1.1 Experimental animals and drugs

Male SD rats of SPF grade (specific pathogen-free grade experimental animals) with a body weight of 210-220 g, purchased from SPF (Beijing) Biotechnology Co., Ltd.

### 4.1.2 Drugs

Papain: 716M022, purchased from Solarbio
Flurbiprofen gel plaster, manufacturer: MIKASA SEIYAKU CO., LTD KAKEGAWA FACTORY, packaging company: Beijing Tide Pharmaceutical Co., Ltd., batch number: 103023-01
L-cysteine: WXBD0897V, Sigma

### 4.2 Experimental method

### 4.2.1 Group settings

This experiment was carried out in 6 groups of rats with each group consisting of 10 rats. The six groups were a normal control group, a model group, a flurbiprofen gel plaster group (a positive drug group), Example 2, Example 5, and Example 7. A blank matrix of the brucine gel plaster preparation was applied to the rats in both the normal control group and the model group on their knee joints.

### 4.2.2 Dosing cycle

The administration lasted for 14 consecutive days, once a day.

### 4.2.3 Establishment of Model

The modeling site was the right knee joint, and the modeling was done on one side.

4% Papain and 0.03 mol/L L-cysteine (mixed at a volume ratio of 2:1) were injected into the joint cavity of each rat to establish the model.

### 4.3 Experimental results

The results of behavioral evaluation showed that the Example groups and the flurbiprofen gel plaster group, compared with the model group, had significantly lower behavioral scores (*p*<0.01, *p*<0.05), the rats in these groups were relieved from the discomfort, thereby enabling the improvement of the walking-on-tiptoe behavior of rats.

The results of X-ray films showed that the Example groups and the flurbiprofen gel plaster group, compared with the model group, had significantly lower X-ray scores (*p*<0.001, *p*<0.01, *p*<0.05), the model rats in these groups were significantly relieved from degenerative changes such as narrowed knee joint cavity, osteophyte and osteosclerosis, thereby indicating significant therapeutic effect on knee osteoarthritis.

The results of pain threshold showed that the Example groups and the flurbiprofen gel plaster group, compared with the model group, had significantly increased pain thresholds (*p*<0.001), thereby indicating significant analgesic effect.

The scores of general observation of articular cartilage showed that the Example groups and the flurbiprofen gel plaster group, compared with the model group, had significantly lower scores in the general observation of articular cartilage (*p*<0.01, *p*<0.05), thereby indicating the effect of protecting cartilage.

The results of histopathological microscopic examination showed that, compared with the model group, significantly delayed progress of the lesion could be seen in all Example groups, while in the flurbiprofen gel plaster group, the progress of the lesion was delayed and there was no significant change.

The experimental data results were shown in the following table.

Results of pharmacodynamic experiment on brucine gel plasters in treatment of knee osteoarthritis in rats

| No. | Group | Dosage (mg/kg) | Behavioral score | X-ray film score | Pain threshold | Score of general observation of articular cartilage |
|---|---|---|---|---|---|---|
| 1 | Normal control group | / | 0.0±0.0 | 0.1±0.3 | 24.9±10.5 | 0.0±0.0 |
| 2 | Model group | / | 2.1±0.6### | 2.1±0.7### | 28.2±8.0# | 3.5±0.5### |
| 3 | Flurbiprofen group | 6.0 | 0.9±0.7^{∗∗} | 1.1±0.6^{∗∗∗} | 52.4±14.7^{∗∗∗} | 2.4±1.0^{∗} |
| 4 | Example 2 | 6.0 | 1.3±0.5^{∗} | 1.3±0.8^{∗} | 59.4±14.1^{∗∗∗} | 2.4±1.0^{∗} |
| 5 | Example 5 | 6.0 | 1.1±0.6^{∗∗} | 1.3±0.5^{∗∗} | 71.7±13.4^{∗∗∗} | 2.2±0.8^{∗∗} |
| 6 | Example 7 | 6.0 | 1.1±0.7^{∗∗} | 1.2±0.6^{∗∗} | 66.3±11.7^{∗∗∗} | 2.3±0.7^{∗∗} |

| | | | | | | |
|---|---|---|---|---|---|---|
| Note: Compared with the control group: ###*p*<0.001, #*p*<0.05; compared with the model group: ^{∗∗}*p*<0.01, ^{∗}*p*<0.05. | | | | | | |

The evaluation criteria of the behavioral score, X-ray film score, pain threshold and score of general observation of articular cartilage were as follows.

### (1) Behavioral score

The measurement was conducted before grouping and at the end of the administration.

Measurement method: the rats were allowed to run on a runway that was 1-meter long and was as wide as an A4 paper, with their rear soles coated with ink paste. Scoring was then performed based on how much the ink paste on the model sole occupied the entire sole.

The scoring criteria were as follows.
0 point: the color of the ink paste on the left sole was the same as that on the right sole;
1 point: <25% of the left sole was darker in color than the right sole;
2 points: 25-50% of the left sole was darker in color than the right sole;
3 points: 50-75% of the left sole was darker in color than the right sole;
4 points: >75% of the left sole was darker in color than the right sole.

### (2) X-ray film score

The measurement was conducted before grouping and at the end of the administration.

Measurement method: a high-frequency X-ray machine was used for measurement. The assessment was made in accordance with the K-L grading system.

The scoring criteria were as follows.
0 point: no change;
1 point: suspicious joint space narrowing (JSN) and osteophytic lipping were seen;
2 points: formation of definite osteophyte (OP) and possible JSN were seen;
3 points: definite JSN, osteosclerosis and possible morphological changes were seen;
4 points: relatively large OP, definite JSN, severe osteosclerosis and bone deformity were seen.

### (3) Measurement of mechanical pain value

The measurement was conducted at the end of the administration.

Measurement method: von Frey test.

### (4) General observation of articular cartilage

The measurement was conducted at the end of the experiment.

Measurement method: After the rats were euthanized, their knee joints and cartilages were observed, and Pelletier scoring was performed.

The scoring criteria were as follows.
0 point: the articular surface was smooth and had normal color and lustre;
1 point: the articular surface was rough, had small cracks, and was gray and dark in color and lustre;
2 points: cartilage defects reached deep to the middle layer of the cartilage;
3 points: ulcer formed on the cartilage surface, and cartilage defects reached deep to the deep layer of the cartilage;
4 points: cartilage denudation occurred and the subchondral bone was exposed.

## Claims

1. A brucine gel plaster, comprising 0.3 wt% to 2 wt% of brucine, 3 wt% to 10 wt% of a framework material, 20 wt% to 50 wt% of a humectant and a cosolvent, 0.1 wt% to 2 wt% of a cross-linking agent and a cross-linking regulator, and water as balance, as well as a backing layer and an anti-adhesive layer; more preferably, the plaster comprising 0.3 wt% to 1 wt% of brucine, 4 wt% to 8 wt% of a framework material, 25 wt% to 40 wt% of a humectant and a cosolvent, 0.3 wt% to 1 wt% of a cross-linking agent and a cross-linking regulator, and water as balance, as well as a backing layer and an anti-adhesive layer.

2. The brucine gel plaster according to claim 1, wherein the framework material is one or more selected from the group consisting of carbomer, sodium alginate, gum arabic, tragacanth, gelatin, starch, xanthan gum, polyacrylic acid, sodium polyacrylate, polyvinyl alcohol, povidone K90, povidone K30, sodium carboxymethylcellulose, polyvinylpyrrolidone, HPMC, alginic acid and its sodium salt, polyacrylic acid cross-linked resin and a partially neutralized product of polyacrylic acid; preferably, the framework material is one or more selected from the group consisting of a partially neutralized product of polyacrylic acid, sodium polyacrylate, povidone K90 and sodium carboxymethylcellulose.

3. The brucine gel plaster according to claim 1, wherein the humectant is one or more selected from the group consisting of ethanol, propylene glycol, glycerin, polyethylene glycol 400 and sorbitol; the cosolvent is one or more selected from the group consisting of ethanol, urea, olive oil, polysorbate 80, polysorbate 60, propylene glycol, DMSO, diethylene glycol monoethyl ether, polyethylene glycol 400, eucalyptus oil and cyclodextrin; preferably, the humectant is one or a combination of two or more selected from the group consisting of glycerin, propylene glycol and sorbitol; and the cosolvent is one or a combination of two or more selected from the group consisting of DMSO, polysorbate 80, polyethylene glycol 400 and cyclodextrin.

4. The brucine gel plaster according to claim 1, wherein the cross-linking agent is one or more selected from the group consisting of aluminum hydroxide, aluminum glycinate, aluminum trichloride, aluminum sulfate, alum, and a complex aluminum salt; the cross-linking regulator is one or more selected from the group consisting of tartaric acid, citric acid, malic acid, edetic acid, and a sodium salt thereof; preferably, the cross-linking agent is one or a combination of two selected from the group consisting of aluminum glycinate and aluminum hydroxide; the crosslinking regulator is one or more selected from the group consisting of tartaric acid, citric acid, edetic acid, and a sodium salt thereof.

5. The brucine gel plaster according to claim 1, wherein the backing layer is selected from non-woven fabric and rayon; the anti-adhesive layer is selected from embossed polypropylene film, polyethylene film, anti-adhesive paper, plastic thin film and hard gauze.

6. The brucine gel plaster according to any one of claims 1 to 5, further comprising 0.05 wt% to 0.075 wt% of an antioxidant, the antioxidant being one or more selected from the group consisting of anhydrous sodium sulfite, sodium metabisulfite, sodium bisulfite, sodium thiosulfate, tert-butyl p-hydroxyanisole, dibutylphenol, vitamin C, vitamin C palmitate, edetic acid and its sodium salt.

7. The brucine gel plaster according to any one of claims 1 to 5, further comprising 0.01 wt% to 0.3 wt% of a bacteriostatic agent, preferably 0.1 wt% to 0.2 wt% of a bacteriostatic agent, the bacteriostatic agent being one or more selected from the group consisting of chlorobutanol, ethylparaben, benzyl alcohol, methylparaben, butylparaben, sorbic acid and its potassium salt, menthol, and benzoic acid and its sodium salt.

8. The brucine gel plaster according to any one of claims 1 to 5, further comprising 0.1 wt% to 10 wt% of a filler, preferably 0.1 wt% to 5 wt% of a filler, the filler being one or more selected from the group consisting of kaolin, diatomaceous earth, bentonite, zinc oxide, titanium dioxide and micro-powder silica gel, preferably, the filler being one or more selected from the group consisting of kaolin, titanium dioxide and micro-powder silica gel.

9. A method for preparing the brucine gel plaster according to any one of claims 1 to 5, the method being selected from the following methods:
method 1, comprising the following steps: (1) preparing a blank matrix that comprises a humectant and a cosolvent, a framework material, a cross-linking agent and a cross-linking regulator, and water in a prescribed amount; (2) dissolving brucine in the cosolvent and mixing with the blank matrix to obtain a brucine paste; and (3) applying the brucine paste on a backing layer and attaching an anti-adhesive layer to obtain a brucine gel plaster, wherein the blank matrix in step (1) further optionally comprises a bacteriostatic agent and an antioxidant;
method 2, comprising the following steps: (1) preparing a brucine organic phase that comprises a humectant and a cosolvent, brucine, a framework material, and a cross-linking agent and a cross-linking regulator; (2) preparing an aqueous matrix that comprises a cross-linking agent and a cross-linking regulator and water; (3) mixing the brucine organic phase with the aqueous matrix to obtain a brucine paste; and (4) applying the brucine paste on a backing layer and attaching an anti-adhesive layer to obtain a brucine gel plaster, wherein the organic phase in step (1) further optionally comprises a bacteriostatic agent and an antioxidant;
method 3, comprising the following steps: (1) preparing an organic phase matrix that comprises a humectant, a framework material, and a cross-linking agent and a cross-linking regulator; (2) preparing a brucine aqueous phase that comprises brucine, a cross-linking agent and a cross-linking regulator, a cosolvent, and water; (3) mixing the organic phase matrix with the brucine aqueous phase to obtain a brucine paste; and (4) applying the brucine paste on a backing layer and attaching an anti-adhesive layer to obtain a brucine gel plaster, wherein the organic phase matrix in step (1) further optionally comprises a bacteriostatic agent and an antioxidant;
method 4, comprising the following steps: (1) preparing an organic phase matrix that comprises a humectant, a framework material, and a cross-linking agent and a cross-linking regulator; (2) preparing a brucine aqueous phase that comprises brucine, a cross-linking regulator, and water; (3) mixing the organic phase matrix with the brucine aqueous phase to obtain a brucine paste; and (4) applying the brucine paste on a backing layer and attaching an anti-adhesive layer to obtain a brucine gel plaster, wherein the organic phase matrix in step (1) further optionally comprises a bacteriostatic agent and an antioxidant.

10. Use of the brucine gel plaster according to any one of claims 1 to 5 in the preparation of a medicament for treating or ameliorating related diseases requiring anti-inflammation and analgesia such as osteoarthritis, rheumatic arthritis, and rheumatoid arthritis.
